# EUROPEAN PATENT APPLICATION

(11) **EP 4 032 543 A1**
(43) Date of publication of application: **27.07.2022**
(21) Application number: 21152539.9
(22) Date of filing: 20.01.2021
(51) Int. Cl.: A61K 38/48, A61P 31/22

(54) **PEPTIDES FROM THE SEQUENCE OF THE COMPLEMENT FACTOR D FOR MEDICAL USE, ESPECIALLY FOR THE TREATMENT OF EBV-ASSOCIATED DISEASES**

(71) Applicant: Fraunhofer-Gesellschaft zur Förderung der angewandten Forschung e.V., 80686 München (DE)
(72) Inventor: SAUER, Eileen., 70569 Stuttgart (DE); BAILER, Susanne., 70569 Stuttgart (DE)
(74) Representative: Wohlfahrt, Jan Günther

(57) **Abstract**

The present invention refers to peptides derived from the complement factor D and their use in the treatment or diagnosis of diseases, e.g. EBV-associated diseases.

## Description

The present invention refers to peptides derived from the complement factor D and their use in the treatment or diagnosis of diseases, e.g. EBV-associated diseases.

The Epstein-Barr virus (EBV) is one of the nine known human herpesvirus types in the herpes family, and is one of the most common viruses in humans. It is best known as the cause of infectious mononucleosis. It is also associated with various non-malignant, premalignant, and malignant Epstein-Barr virus-associated lymphoproliferative diseases such as Burkitt lymphoma, PTLD, and Hodgkin's lymphoma; non-lymphoid malignancies such as gastric cancer and nasopharyngeal carcinoma; and conditions associated with human immunodeficiency virus such as hairy leukoplakia and central nervous system lymphomas. EBV can infect both B cells and epithelial cells.

In many EBV-associated tumours the tumour cells are 100 % EBV-positive. On this basis different therapeutic strategies were developed, which use the EBV-genome present in the tumour cells. One of these strategies is the lytic induction therapy, also known as cytolytic virus activation therapy (CLVA-therapy). This therapy is a combination therapy combining lytic EBV-induction and antiviral treatment for the inhibition of the viral DNA replication. In a first step the lytic EBV-replication is induced by proviral substances, i.e. lytic inducer agents like valproic acid, phorbol 12-myristate 13-acetate (PMA) or romidepsin, so that the expression of lytic EBV-genes is reactivated. Due to this expression of viral lytic gene products the recognition of tumour cells by the immune system is triggered, so that cytotoxic T-cells against lytic EBV-proteins are activated. Furthermore, EBV-positive cells get sensitized for substances with antiviral activity, e.g. Ganciclovir. In a number of studies it was shown that the induction of the lytic EBV-cycle can also provoke in the absence of productive EBV-virus particles an apoptotic cell death in different kinds of EBV-infected cell lines. Taken together, the proviral substances can lead to a lytic EBV-induction, so that the tumour cells express lytic EBV-proteins. This results advantageously in an immune recognition, in a sensitization for antiviral therapeutics and/or in direct apoptosis and finally to the selective elimination of EBV-positive cells.

However, the presently known proviral active agents are often also toxic for healthy cells and/or are not sufficiently efficient for the induction of the EBV lytic cycle. Accordingly, the non-toxicity and the efficiency of the proviral compounds is a key feature for a successful lytic induction therapy.

The problem underlying the present invention is the provision of better proviral substances, i.e. especially proviral substances which are less toxic or non-toxic for healthy cells and which induce at the same time the expression of lytic EBV-proteins in a sufficient amount.

The problem underlying the present invention is solved by the provision of a peptide according to claim 1.

The problem underlying the present invention is solved by the provision of a peptide comprising a sequence of at least 5 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use in the treatment or diagnosis of a disease selected from the group consisting of Epstein-Barr virus associated diseases, B-cell associated diseases, epithelial cell associated diseases, T-cell associated diseases and combinations thereof or for the use in combination therapies for treating a tumour or for use as co-factor in T-cell therapy or for use in lytic induction therapy.

The problem underlying the present invention is solved by the provision of a peptide comprising a sequence of at least 7 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use in the treatment or diagnosis of a disease selected from the group consisting of Epstein-Barr virus associated diseases, B-cell associated diseases, epithelial cell associated diseases, T-cell associated diseases and combinations thereof or for the use in combination therapies for treating a tumour or for use as co-factor in T-cell therapy or for use in lytic induction therapy.

The problem underlying the present invention is solved by the provision of a peptide comprising a sequence of at least 10 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use in the treatment or diagnosis of a disease selected from the group consisting of Epstein-Barr virus associated diseases, B-cell associated diseases, epithelial cell associated diseases, T-cell associated diseases and combinations thereof or for the use in combination therapies for treating a tumour or for use as co-factor in T-cell therapy or for use in lytic induction therapy.

Preferably the peptide or derivatives, and variants and fragments of the peptide are used as proviral substance or compound, i.e. they are lytic inducer agents and can induce the lytic cycle of a virus, preferably an EBV-virus in EBV-positive cells, preferably EBV-positive human cells.

The problem underlying the present invention is solved by the provision of a peptide comprising a sequence of at least 5 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use as proviral substance or compound.

The problem underlying the present invention is solved by the provision of a peptide comprising a sequence of at least 7 amino acids from the sequence of the complement factor D or derivatives, and variants and fragments of the peptide or a pharmaceutically and/or physiologically acceptable salt thereof, wherein said derivatives, fragments and variants have at least 80 % sequence identity with said peptide, for use as proviral substance or compound.

The problem underlying the present invention is solved by the provision of a peptide comprising a sequence of at least 10 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use as proviral substance or compound.

The inventors of the present invention found that surprisingly the complement factor D protein and fragments thereof show a proviral activity. It was shown that peptides derived from the sequence of the human complement factor D can induce the expression of lytic EBV-genes and EBV-proteins in human EBV-positive tumour cells. Furthermore, the use of the complement factor D and fragments thereof as lytic inducer agent, i.e. as proviral substance or compound, has the advantage that according peptides are endogenous peptides being present in the blood and are therefore biocompatible and non-toxic, especially compared to the non-natural or exogenous proviral compounds used in the state of the art.

The complement factor D (EC3.4.21.46), also known as C3 proactivator convertase, properdin factor D esterase and adipsin, is a protein which in humans is encoded by the CFD-gene. The complement factor D protein (CFD) is involved in the alternative complement pathway of the complement system where it cleaves factor B. The CFD-protein is a serin protease that is on one side a component of the alternative complement pathway, best known for its role in humoral suppression of infectious agents and is also secreted by adipocytes into the blood stream.

In the context of the present invention, the term "peptide" refers to oligopeptides, polypeptides and proteins. Accordingly, a peptide comprising a sequence of at least 5, 7 or 10 amino acids from the sequence of the complement factor D comprises at least 5, 7 or 10 amino acids, but includes also longer peptides up to the whole complement factor D protein. Accordingly, the peptide according to the present invention can be the whole complement factor D protein or a part thereof, wherein the part thereof has at least 10 amino acids. Accordingly the peptide can have from 10 amino acids up to the amino acid number of the whole complement factor D protein, wherein every amino acid number in between is also disclosed.

The invention does not only refer to an according peptide, but also to pharmaceutically and/or physiologically acceptable salts of the peptide, especially a pharmaceutically acceptable salt. The peptide can be present in any suitable formulation, e.g. in dissolved form, in solid form or as salt.

The peptide can be present in a peptide mixture. In a preferred embodiment the peptide is not in a mixture of peptides, especially in a mixture of a number of peptides of unknown sequence, but is individualized, purified and/or homogenized. Of course, the peptide can be present also in a mixture with specific other peptides, especially specific other peptides according to the present invention. For example, a specific mixture of 2, 3, 4, 5, 6, 7, 8, 9, 10 or 11 to 50, or 11 to 100 peptides according to the present invention can be provided. In a preferred embodiment the peptide is purified and/or homogenized. In a preferred embodiment the peptide is used as a single peptide, i.e. as an individualized peptide.

Preferably the complement factor D is similar to the human complement factor D.

In a preferred embodiment the complement factor D has a homology to the human complement factor D of at least 70 %, more preferably at least 80 %, even more preferably at least 85 %, even more preferably at least 90 %, even more preferably 95 %, even more preferably at least 98 %, most preferably at least 99 %.

In a preferred embodiment the peptide comprises a sequence of at least 10 amino acids from the sequence of the complement factor D of a mammal, or derivatives, and variants and fragments of the peptide, wherein said derivatives, fragments and variants have at least 80 % sequence identity with said peptide.

Preferably the complement factor D of a mammal is similar to the human complement factor D.

In a preferred embodiment the mammalian complement factor D is a complement factor D from a primate, especially a complement factor D of a rhesus macaque, a cynomolgus macaque or a human.

In a preferred embodiment the peptide comprises a sequence of at least 10 amino acids from the sequence of the human complement factor D, or derivatives, and variants and fragments of the peptide, wherein said derivatives, fragments and variants have at least 80 % sequence identity with said peptide.

The human complement factor D protein including the signal peptide and the propeptide has following sequence (SEQ ID No. 5):

MHSWERLAVLVLLGAAACAAPPRGRILGGREAEAHARPYMASVQLNGAHLCGGVLVA
EQWVLSAAHCLEDAADGKVQVLLGAHSLSQPEPSKRLYDVLRAVPHPDSQPDTIDHDL
LLLQLSEKATLGPAVRPLPWQRVDRDVAPGTLCDVAGWGIVNHAGRRPDSLQHVLLPV
LDRATCNRRTHHDGAITERLMCAESNRRDSCKGDSGGPLVCGGVLEGVVTSGSRVCGN
RKKPGIYTRVASYAAWIDSVLA

The chain of the human complement factor D protein without the signal peptide and the propeptide has following sequence (AA 26-253; SEQ ID No. 6):

ILGGREAEAHARPYMASVQLNGAHLCGGVLVAEQWVLSAAHCLEDAADGKVQVLLG
AHSLSQPEPSKRLYDVLRAVPHPDSQPDTIDHDLLLLQLSEKATLGPAVRPLPWQRVDR

DVAPGTLCDVAGWGIVNHAGRRPDSLQHVLLPVLDRATCNRRTHHDGAITERLMCAES
NRRDSCKGDSGGPLVCGGVLEGVVTSGSRVCGNRKKPGIYTRVASYAAWIDSVLA

In a preferred embodiment the peptide comprises a sequence of at least 10 amino acids from SEQ ID No. 5 or a pharmaceutically and/or physiologically acceptable salt thereof. In a more preferred embodiment the peptide comprises a sequence of at least 10 amino acids from SEQ ID No. 6 or a pharmaceutically and/or physiologically acceptable salt thereof. The invention refers also to according derivatives, variants and fragments of a peptide comprising a sequence of at least 10 amino acids from SEQ ID No. 5 or SEQ ID No. 6, wherein said derivatives, fragments and variants have at least 80 % sequence identity with said peptide, more preferably have at least 90 % sequence identity with said peptide, most preferably have at least 95 % sequence identity with said peptide or a pharmaceutically and/or physiologically acceptable salt thereof.

In a preferred embodiment the peptide comprises a sequence of at least 10 amino acids from SEQ ID No. 1 or derivatives, variants and fragments of the peptide, wherein said derivatives, fragments and variants have at least 80 % sequence identity with said peptide or a pharmaceutically and/or physiologically acceptable salt thereof.

SEQ ID No. 1:
PAVRPLPWQRVDRDVAPGTLCDVAGWGIVNHAGRRPDSLQHVLLPVLDRATCNRRTH
HDGAITERLMCAESNRRDSCKGDSGGPLVCGGVLEGVVTSGSRV

In a preferred embodiment the peptide comprises a sequence of at least 10 amino acids from SEQ ID No. 2 or derivatives, variants and fragments of the peptide, wherein said derivatives, fragments and variants have at least 80 % sequence identity with said peptide or a pharmaceutically and/or physiologically acceptable salt thereof.

SEQ ID No. 2:
VDRDVAPGTLCDVAGWGIVNHAGRRPDSLQHVLLPVLDRATCNRRTHHDGAITERLM
CAESNRRDSCKGDSGGPLVCGGVL

The inventors found two very specific fragments of the complement factor D protein being suitable preferred embodiments of the peptides according to the present invention:
SEQ ID No. 3: LQLSEKATLGPAVRPLPWQRVDRDVAPGTLCDVAGW

SEQ ID No. 3 is a 36mer consisting of the amino acids 118-153 of the complement factor D protein.

SEQ ID No. 4:
GIVNHAGRRPDSLQHVLLPVLDRATCNRRTHHDGAITERLMCAESNRRDSCKGDSGGP
LVCGGVLEGVVTSGSRVCGNRKKPGIY

SEQ ID No. 4 is a 85mer consisting of the amino acids 154-238 of the complement factor D protein.

In a preferred embodiment the peptide comprises a sequence of at least 10 amino acids from SEQ ID No. 3 or derivatives, variants and fragments of the peptide, wherein said derivatives, fragments and variants have at least 80 % sequence identity with said peptide or a pharmaceutically and/or physiologically acceptable salt thereof.

In a preferred embodiment the peptide comprises a sequence of at least 10 amino acids from SEQ ID No. 4 or derivatives, variants and fragments of the peptide, wherein said derivatives, fragments and variants have at least 80 % sequence identity with said peptide or a pharmaceutically and/or physiologically acceptable salt thereof.

In a preferred embodiment the peptide is the sequence SEQ ID No. 3. In a preferred embodiment the peptide is the sequence SEQ ID No. 4. In a preferred embodiment there is a mixture of a peptide with the sequence SEQ ID No. 3 and a peptide with the sequence SEQ ID No. 4.

In a preferred embodiment the peptide comprises a sequence of at least 10 amino acids from SEQ ID No. 7 or derivatives, variants and fragments of the peptide, wherein said derivatives, fragments and variants have at least 80 % sequence identity with said peptide or a pharmaceutically and/or physiologically acceptable salt thereof.

SEQ ID No. 7 is the sequence of SEQ ID No. 3 and SEQ ID No. 4 together:

LQLSEKATLGPAVRPLPWQRVDRDVAPGTLCDVAGWGIVNHAGRRPDSLQHVLLPVLD
RATCNRRTHHDGAITERLMCAESNRRDSCKGDSGGPLVCGGVLEGVVTSGSRVCGNRK
KPGIY

In a preferred embodiment the peptide comprises a sequence of at least 10 amino acids from SEQ ID No. 8 or derivatives, variants and fragments of the peptide, wherein said derivatives, fragments and variants have at least 80 % sequence identity with said peptide or a pharmaceutically and/or physiologically acceptable salt thereof.

SEQ ID No. 8:
ILGGREAEAHARPYMASVQLNGAHLCGGVLVAEQWVLSAAHCLEDAADGKVQVLLG
AHSLSQPEPSKRLYDVLRAVPHPDSQPDTIDHDLLL

In a preferred embodiment the derivatives, fragments and variants have at least 90 % sequence identity, more preferably at least 95 % sequence identity, more preferably at least 98 % sequence identity with said peptide.

The derivatives, variants and fragments of the peptide can also be amidated, acetylated sulfated, phosphorylated, glycosylated, oxidized or polyethylene glycol-modified derivatives. The derivatives, variants and fragments of the peptide can also be variants and fragments obtained by a conservative exchange and/or deletions of at least one amino acid, and/or variants which contain from 1 to 10 additional amino acids at the N and/or C termini of the peptides.

The invention refers also to derivatives, variants and fragments of the peptide can also be variants and fragments obtained by a conservative exchange of at least one amino acid of the peptide. Such conservative exchanges are especially:
An exchange of Glycine, Alanine, Valine, Leucine, Isoleucine by one of the other aliphatic amino acids Glycine, Alanine, Valine, Leucine, Isoleucine.

An exchange of Serine, Cysteine, Selenocysteine, Threonine, Methionine by one of the other hydroxyl or sulfur/selenium-containing amino acids Serine, Cysteine, Selenocysteine, Threonine, Methionine.

An exchange of Phenylalanine, Tyrosine, Tryptophan by one of the other aromatic amino acids Phenylalanine, Tyrosine, Tryptophan.

An exchange of Histidine, Lysine, Arginine by one of the other basic amino acids Histidine, Lysine, Arginine.

An exchange of Aspartate, Glutamate, Asparagine, Glutamine by one of the other Acidic and their amides Aspartate, Glutamate, Asparagine, Glutamine.

The skilled person knows how to exchange amino acids in a peptide sequence in a conservative or non-conservative manner and to test the function of the resulting peptide.

Preferred embodiments relate to variants which contain from 1 to 5 or 1, 2 or 3 additional amino acids at the N and /or C termini of the peptides.

Preferred embodiments relate to variants which contain from 1 to 1500 or from 1 to 1000 or from 1 to 500 or from 1 to 300 or from 1 to 100 additional amino acids at the N and /or C termini of the peptides.

The peptide can also contain one or more disulfide bridges or can be connected to other peptides, including proteins, via disulfide bridges.

In one embodiment also at least two peptides according to the invention, e.g. the peptide with SEQ ID No. 3 and the peptide with SEQ ID No. 4, can be connected via at least one disulfide bridge.

The peptide can also be associated with tag, e.g. a protein-tag or a peptide-tag. Accordingly, the peptide can have at the N and /or C termini of the peptide a tag, wherein the tag comprises at least 1 amino acid up to more then 100 amino acids, which can for example be a functional protein. The peptide can also be fused to another peptide or protein or can be part of another protein.

In a preferred embodiment the peptide derivatives, variants and fragments of the peptide or the pharmaceutically and/or physiologically acceptable salt thereof according to the present invention have a lytic inducer activity on EBV-positive cells, preferably EBV-positive tumour cells.

Preferably the peptides, derivatives, variants and fragments of the peptide or the pharmaceutically and/or physiologically acceptable salt thereof according to the present invention have a lytic inducer activity in a cellular infection assay.

The skilled person knows how to test the lytic inducer activity of a peptide.

In a preferred embodiment the peptide comprises at least 11, more preferably at least 12, more preferably at least 13, more preferably at least 14, more preferably at least 15, more preferably at least 16, more preferably at least 17, more preferably at least 18, more preferably at least 19 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 20 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 25 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 30 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 35 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 36 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide comprises at least 40, more preferably at least 50, more preferably at least 60, more preferably at least 70, more preferably at least 80, more preferably at least 16, more preferably at least 17, more preferably at least 18, more preferably at least 19 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof.

In a preferred embodiment the peptide, the derivative, the variant or the fragment is for use in the treatment of an Epstein-Barr virus associated disease.

In a preferred embodiment the Epstein-Barr virus associated disease is an Epstein-Barr virus associated tumor.

In a preferred embodiment the peptide, the derivative, the variant or the fragment is for use as Epstein-Barr virus inductor. In a preferred embodiment the peptide, the derivative, the variant or the fragment is for use as a lytic Epstein-Barr virus inductor.

In a preferred embodiment the peptide, the derivative, the variant or the fragment is for use in a lytic induction therapy.

The present invention refers also to a mixture of at least two different peptides, each comprising a sequence of at least 10 amino acids from the sequence of the complement factor D or derivatives, and variants and fragments of the peptides, wherein said derivatives, fragments and variants have at least 80 % sequence identity with said peptides.

In a preferred embodiment the at least two different peptides are each a peptide according to the present invention.

The present invention refers also to a pharmaceutical composition or a pharmaceutical formulation comprising at least one peptide according to the present invention and a pharmaceutically acceptable carrier. The pharmaceutically acceptable carrier can be any suitable pharmaceutically acceptable carrier, e.g. a liquid carrier like water or alcohol or a solid carrier like binders, e.g. a sugar or a bulk excipient.

The peptide can be present in any suitable formulation, e.g. in dissolved form, in solid form or as salt.

In a preferred embodiment the pharmaceutical composition or pharmaceutical formulation is an infusion, tablet, ointment, spray, capsule.

The present invention refers also to the use of a peptide comprising a sequence of at least 10 amino acids from the sequence of the complement factor D or derivatives and variants and fragments of the peptide, wherein said derivatives, fragments and variants have at least 80 % sequence identity with said peptide, for the manufacturing of a medicament for the treatment or diagnosis of a disease, especially a disease outlined in the present disclosure.

The present invention also refers to a peptide comprising a sequence of at least 10 amino acids from the sequence of the complement factor D or derivatives and variants and fragments of the peptide, wherein said derivatives, fragments and variants have at least 80 % sequence identity with said peptide as a product itself, especially if this peptide is homogenized, purified, extracted and/or individualized. The present invention also refers to a pharmaceutically and/or physiologically acceptable salt of such a peptide.

The present invention refers also to the use of a peptide comprising a sequence of at least 10 amino acids from the sequence of the complement factor D or derivatives, and variants and fragments of the peptide, wherein said derivatives, fragments and variants have at least 80 % sequence identity with said peptide, for the in vitro diagnosing of a disease selected from the group selected of B-cell associated diseases, epithelial cell associated diseases, T-cell associated diseases, Epstein-Barr virus associated diseases or of a tumour and/or as a marker.

The present invention refers also to a peptide comprising a sequence of at least 10 amino acids from the sequence of the complement factor D or derivatives, and variants and fragments of the peptide, wherein said derivatives, fragments and variants have at least 80 % sequence identity with said peptide, wherein the peptide is a marker.

Further preferred embodiments are outlined in the dependent claims.

It should be noted that embodiments and features described in the context of one of the aspects of the present invention also apply to the other aspects of the invention. It should be noted that embodiments and features described in the context of the peptide according to the present invention refer also to derivatives, variants and fragments of the peptide, as well as to pharmaceutically and/or physiologically acceptable salts of the peptide. It should be also noted that the reference to a peptide according to the present invention refers both to an according single peptide or to a mixture of peptides, especially to a mixture of different peptides according to the present invention and a purified single peptide or peptide mixture.

All patent and non-patent references cited in the present application are hereby incorporated by reference in their entirety. Preferred embodiments of the present invention are described also in the following in the non-limiting examples and the figure.

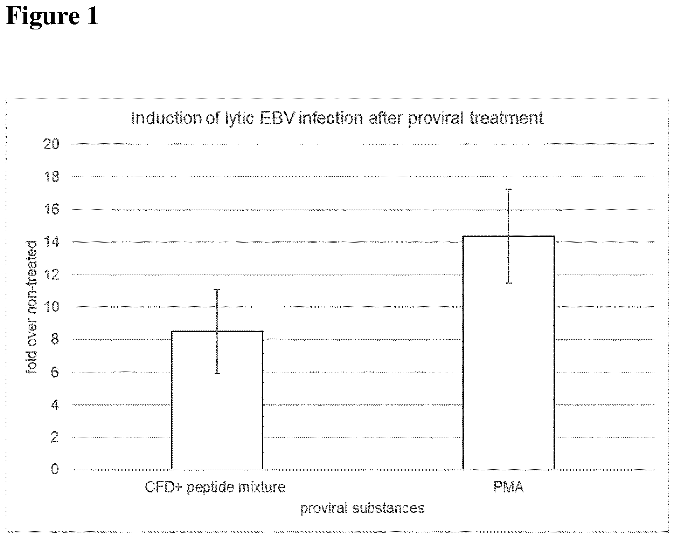

- Figure 1: shows the relative induction of lytic EBV-infection after proviral treatment with peptides of complement factor D contained in human peptide mixture in comparison with PMA.

### Examples

### 1. Screening for endogenous proviral peptides

For the identification of suitable proviral peptides for the lytic induction therapy, a screening for active compounds in a peptide library was used. Endogenous peptides can be a source of immune modulating substances, which have a potential high biocompatibility and a low toxicity. Aim of the screening was the identification of human peptides with proviral properties, which induce the lytic EBV-cycle and reactivate the latent EBV-infect in an efficient manner. The screening comprised an iterative process of screening and chromatographic separation until the biological active compound is present in a purified form and can be identified.

For the screening of this peptide library a specific reporter cell line for the lytic EBV-reactivation was used, which allowed the detection and quantification of the EBV-reactivation via a reporter protein.

The screening revealed two peptides, which induced the production of the reporter protein. The two peptides were analysed.

The first peptide had the sequence (SEQ ID No. 3):
LQLSEKATLGPAVRPLPWQRVDRDVAPGTLCDVAGW

The second peptide had the sequence (SEQ ID No. 4):

GIVNHAGRRPDSLQHVLLPVLDRATCNRRTHHDGAITERLMCAESNRRDSCKGDSGGP
LVCGGVLEGVVTSGSRVCGNRKKPGIY

Both sequences are part of the sequence of the human complement factor D as shown in SEQ ID No. 5 and SEQ ID No. 6. Accordingly, the screening revealed two peptides comprising a sequence of at least 10 amino acids from the sequence of the human complement factor D, which showed the ability to induce the EBV-reactivation and the switch of the latent to the lytic cycle.

### 2. Induction of lytic EBV-infection after proviral treatment

The relative induction of the EBV lytic cycle in latently EBV-infected Raji-reporter cells was tested using the human peptide mixture comprising the two complement factor D derived peptide sequences SEQ ID No. 3 and SEQ ID No. 4 (CFD+ peptide mixture) in comparison to the known exogenous proviral substance phorbol-12-myristate13-acetate (PMA), each related to the untreated control. The EBV-positive Raji-reporter cells were treated with the human peptide mixture or with the 20 ng/ml PMA. In a control, cells were not treated with a proviral substance. After 24 hours induction the lytic EBV-infection was compared between treated and non-treated cells via expression of the lytic EBV-immediate early protein ZEBRA. As shown in Figure 1, not only the PMA treatment, but also the treatment with the human peptide mixture containing the two complement factor D derived peptides, resulted in the induction of lytic EBV-infection. For this result it has to be taken into account that the human peptide mixture was used and not the pure peptides. However, even the human peptide mixture resulted in an induction not much lower than the induction by PMA. Without being bound to the theory, it can be assumed that the pure peptides will result in an even better induction.

### 3. Further purification

The human peptide mixture of example 2 was further purified using the iterative process of screening and chromatographic separation as in example 1. In the end the two peptides were the only peptides in the human peptide mixture used in example 2, which resulted in a positive signal in the specific reporter cell line of example 1. This shows, that the two complement factor D derived peptides are the active compound in the human peptide mixture of example 2.

The peptides according to the present invention are accordingly a biogenic and endogenous substance, which has an induction capacity comparable to exogenous active agents, which are often toxic.

## Claims

1. A peptide comprising a sequence of at least 10 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for use in the treatment or diagnosis of a disease selected from the group consisting of Epstein-Barr virus associated diseases, B-cell associated diseases, epithelial cell associated diseases, T-cell associated diseases and combinations thereof or for the use in combination therapies for treating a tumour or for use as co-factor in T-cell therapy or for use in lytic induction therapy.

2. A peptide according to claim 1, wherein the peptide comprises a sequence of at least 10 amino acids from the sequence of the complement factor D of a mammal, or derivatives, and variants and fragments of the peptide, wherein said derivatives, fragments and variants have at least 80 % sequence identity with said peptide or a pharmaceutically and/or physiologically acceptable salt thereof.

3. A peptide according to claim 1 or according to claim 2, wherein the peptide comprises a sequence of at least 10 amino acids from SEQ ID No. 6 or derivatives, variants and fragments of the peptide, wherein said derivatives, fragments and variants have at least 80 % sequence identity with said peptide or a pharmaceutically and/or physiologically acceptable salt thereof.

4. A peptide according to any of the preceding claims, wherein the peptide comprises a sequence of at least 10 amino acids from SEQ ID No. 7 or derivatives, variants and fragments of the peptide, wherein said derivatives, fragments and variants have at least 80 % sequence identity with said peptide or a pharmaceutically and/or physiologically acceptable salt thereof.

5. A peptide according to any of the preceding claims, wherein the peptide comprises a sequence of at least 10 amino acids from SEQ ID No. 3 or derivatives, variants and fragments of the peptide, wherein said derivatives, fragments and variants have at least 80 % sequence identity with said peptide or a pharmaceutically and/or physiologically acceptable salt thereof.

6. A peptide according to any of the preceding claims, wherein the peptide comprises a sequence of at least 10 amino acids from SEQ ID No. 4 or derivatives, variants and fragments of the peptide, wherein said derivatives, fragments and variants have at least 80 % sequence identity with said peptide or a pharmaceutically and/or physiologically acceptable salt thereof.

7. A derivative, a variant or a fragment of a peptide according to any of the preceding claims, wherein said derivatives, fragments and variants have at least 90 % sequence identity, more preferably at least 95 % sequence identity with said peptide or a pharmaceutically and/or physiologically acceptable salt thereof.

8. A peptide according to any of the preceding claims, wherein the peptide comprises at least 25 amino acids from the sequence of the complement factor D or derivatives, and variants and fragments of the peptide, wherein said derivatives, fragments and variants have at least 80 % sequence identity with said peptide or a pharmaceuticalyl and/or physiologically acceptable salt thereof.

9. A peptide, a derivative, a variant or a fragment or a pharmaceutically and/or physiologically acceptable salt thereof according to any of the preceding claims, for use in the treatment of an Epstein-Barr virus associated disease or for use as Epstein-Barr virus inductor.

10. A mixture of at least two different peptides, each comprising a sequence of at least 10 amino acids from the sequence of the complement factor D or derivatives, and variants and fragments of the peptides, wherein said derivatives, fragments and variants have at least 80 % sequence identity with said peptides or pharmaceutically and/or physiologically acceptable salts thereof.

11. The mixture according to claim 10, wherein the at least two different peptides or derivatives, and variants and fragments of the peptides, wherein said derivatives, fragments and variants have at least 80 % sequence identity with said peptides or pharmaceutically and/or physiologically acceptable salts thereof are each a peptide according to claims 1 to 9.

12. A pharmaceutical composition comprising at least one peptide or derivatives, and variants and fragments of the peptides, wherein said derivatives, fragments and variants have at least 80 % sequence identity with said peptides or a pharmaceutically and/or physiologically acceptable salt thereof according to any of the preceding claims and a pharmaceutically and/or physiologically acceptable carrier.

13. A pharmaceutical composition according to claim 12, wherein the pharmaceutical composition is an infusion, tablet, ointment, spray, capsule.

14. Use of a peptide comprising a sequence of at least 10 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, for the in vitro diagnosing of a disease selected from the group consisting of B-cell associated diseases, T-cell associated diseases epithelial cell associated diseases, Epstein-Barr virus associated diseases or of a tumour and/or as a marker.

15. A peptide comprising a sequence of at least 10 amino acids from the sequence of the complement factor D or derivatives, and variants and fragments of the peptide, wherein said derivatives, fragments and variants have at least 80 % sequence identity with said peptide or a pharmaceutically and/or physiologically acceptable salt thereof, wherein the peptide is a marker.


SEQUENCE LISTING

<110>  Fraunhofer-Gesellschaft zur Förderung der angewandten
       Forschung e.V.

<120>  Peptides from the sequence of the complement factor D for medical
        use, especially for the treatment of EBV-associated diseases

<130>  208700

<160>  8

<170>  PatentIn version 3.5

<210>  1
<211>  101
<212>  PRT
<213>  Homo sapiens

<400>  1

Pro Ala Val Arg Pro Leu Pro Trp Gln Arg Val Asp Arg Asp Val Ala
1               5                   10                  15

Pro Gly Thr Leu Cys Asp Val Ala Gly Trp Gly Ile Val Asn His Ala
            20                  25                  30

Gly Arg Arg Pro Asp Ser Leu Gln His Val Leu Leu Pro Val Leu Asp
        35                  40                  45

Arg Ala Thr Cys Asn Arg Arg Thr His His Asp Gly Ala Ile Thr Glu
    50                  55                  60

Arg Leu Met Cys Ala Glu Ser Asn Arg Arg Asp Ser Cys Lys Gly Asp
65                  70                  75                  80

Ser Gly Gly Pro Leu Val Cys Gly Gly Val Leu Glu Gly Val Val Thr
                85                  90                  95

Ser Gly Ser Arg Val
            100

<210>  2
<211>  81
<212>  PRT
<213>  Homo sapiens

<400>  2

Val Asp Arg Asp Val Ala Pro Gly Thr Leu Cys Asp Val Ala Gly Trp
1               5                   10                  15

Gly Ile Val Asn His Ala Gly Arg Arg Pro Asp Ser Leu Gln His Val
            20                  25                  30

Leu Leu Pro Val Leu Asp Arg Ala Thr Cys Asn Arg Arg Thr His His
        35                  40                  45

Asp Gly Ala Ile Thr Glu Arg Leu Met Cys Ala Glu Ser Asn Arg Arg
    50                  55                  60

Asp Ser Cys Lys Gly Asp Ser Gly Gly Pro Leu Val Cys Gly Gly Val
65                  70                  75                  80

Leu

<210>  3
<211>  36
<212>  PRT
<213>  Homo sapiens

<400>  3

Leu Gln Leu Ser Glu Lys Ala Thr Leu Gly Pro Ala Val Arg Pro Leu
1               5                   10                  15

Pro Trp Gln Arg Val Asp Arg Asp Val Ala Pro Gly Thr Leu Cys Asp
            20                  25                  30

Val Ala Gly Trp
        35

<210>  4
<211>  85
<212>  PRT
<213>  Homo sapiens

<400>  4

Gly Ile Val Asn His Ala Gly Arg Arg Pro Asp Ser Leu Gln His Val
1               5                   10                  15

Leu Leu Pro Val Leu Asp Arg Ala Thr Cys Asn Arg Arg Thr His His
            20                  25                  30

Asp Gly Ala Ile Thr Glu Arg Leu Met Cys Ala Glu Ser Asn Arg Arg
        35                  40                  45

Asp Ser Cys Lys Gly Asp Ser Gly Gly Pro Leu Val Cys Gly Gly Val
    50                  55                  60

Leu Glu Gly Val Val Thr Ser Gly Ser Arg Val Cys Gly Asn Arg Lys
65                  70                  75                  80

Lys Pro Gly Ile Tyr
                85

<210>  5
<211>  253
<212>  PRT
<213>  Homo sapiens

<400>  5

Met His Ser Trp Glu Arg Leu Ala Val Leu Val Leu Leu Gly Ala Ala
1               5                   10                  15

Ala Cys Ala Ala Pro Pro Arg Gly Arg Ile Leu Gly Gly Arg Glu Ala
            20                  25                  30

Glu Ala His Ala Arg Pro Tyr Met Ala Ser Val Gln Leu Asn Gly Ala
        35                  40                  45

His Leu Cys Gly Gly Val Leu Val Ala Glu Gln Trp Val Leu Ser Ala
    50                  55                  60

Ala His Cys Leu Glu Asp Ala Ala Asp Gly Lys Val Gln Val Leu Leu
65                  70                  75                  80

Gly Ala His Ser Leu Ser Gln Pro Glu Pro Ser Lys Arg Leu Tyr Asp
                85                  90                  95

Val Leu Arg Ala Val Pro His Pro Asp Ser Gln Pro Asp Thr Ile Asp
            100                 105                 110

His Asp Leu Leu Leu Leu Gln Leu Ser Glu Lys Ala Thr Leu Gly Pro
        115                 120                 125

Ala Val Arg Pro Leu Pro Trp Gln Arg Val Asp Arg Asp Val Ala Pro
    130                 135                 140

Gly Thr Leu Cys Asp Val Ala Gly Trp Gly Ile Val Asn His Ala Gly
145                 150                 155                 160

Arg Arg Pro Asp Ser Leu Gln His Val Leu Leu Pro Val Leu Asp Arg
                165                 170                 175

Ala Thr Cys Asn Arg Arg Thr His His Asp Gly Ala Ile Thr Glu Arg
            180                 185                 190

Leu Met Cys Ala Glu Ser Asn Arg Arg Asp Ser Cys Lys Gly Asp Ser
        195                 200                 205

Gly Gly Pro Leu Val Cys Gly Gly Val Leu Glu Gly Val Val Thr Ser
    210                 215                 220

Gly Ser Arg Val Cys Gly Asn Arg Lys Lys Pro Gly Ile Tyr Thr Arg
225                 230                 235                 240

Val Ala Ser Tyr Ala Ala Trp Ile Asp Ser Val Leu Ala
                245                 250

<210>  6
<211>  228
<212>  PRT
<213>  Homo sapiens

<400>  6

Ile Leu Gly Gly Arg Glu Ala Glu Ala His Ala Arg Pro Tyr Met Ala
1               5                   10                  15

Ser Val Gln Leu Asn Gly Ala His Leu Cys Gly Gly Val Leu Val Ala
            20                  25                  30

Glu Gln Trp Val Leu Ser Ala Ala His Cys Leu Glu Asp Ala Ala Asp
        35                  40                  45

Gly Lys Val Gln Val Leu Leu Gly Ala His Ser Leu Ser Gln Pro Glu
    50                  55                  60

Pro Ser Lys Arg Leu Tyr Asp Val Leu Arg Ala Val Pro His Pro Asp
65                  70                  75                  80

Ser Gln Pro Asp Thr Ile Asp His Asp Leu Leu Leu Leu Gln Leu Ser
                85                  90                  95

Glu Lys Ala Thr Leu Gly Pro Ala Val Arg Pro Leu Pro Trp Gln Arg
            100                 105                 110

Val Asp Arg Asp Val Ala Pro Gly Thr Leu Cys Asp Val Ala Gly Trp
        115                 120                 125

Gly Ile Val Asn His Ala Gly Arg Arg Pro Asp Ser Leu Gln His Val
    130                 135                 140

Leu Leu Pro Val Leu Asp Arg Ala Thr Cys Asn Arg Arg Thr His His
145                 150                 155                 160

Asp Gly Ala Ile Thr Glu Arg Leu Met Cys Ala Glu Ser Asn Arg Arg
                165                 170                 175

Asp Ser Cys Lys Gly Asp Ser Gly Gly Pro Leu Val Cys Gly Gly Val
            180                 185                 190

Leu Glu Gly Val Val Thr Ser Gly Ser Arg Val Cys Gly Asn Arg Lys
        195                 200                 205

Lys Pro Gly Ile Tyr Thr Arg Val Ala Ser Tyr Ala Ala Trp Ile Asp
    210                 215                 220

Ser Val Leu Ala
225

<210>  7
<211>  121
<212>  PRT
<213>  Homo sapiens

<400>  7

Leu Gln Leu Ser Glu Lys Ala Thr Leu Gly Pro Ala Val Arg Pro Leu
1               5                   10                  15

Pro Trp Gln Arg Val Asp Arg Asp Val Ala Pro Gly Thr Leu Cys Asp
            20                  25                  30

Val Ala Gly Trp Gly Ile Val Asn His Ala Gly Arg Arg Pro Asp Ser
        35                  40                  45

Leu Gln His Val Leu Leu Pro Val Leu Asp Arg Ala Thr Cys Asn Arg
    50                  55                  60

Arg Thr His His Asp Gly Ala Ile Thr Glu Arg Leu Met Cys Ala Glu
65                  70                  75                  80

Ser Asn Arg Arg Asp Ser Cys Lys Gly Asp Ser Gly Gly Pro Leu Val
                85                  90                  95

Cys Gly Gly Val Leu Glu Gly Val Val Thr Ser Gly Ser Arg Val Cys
            100                 105                 110

Gly Asn Arg Lys Lys Pro Gly Ile Tyr
        115                 120

<210>  8
<211>  92
<212>  PRT
<213>  Homo sapiens

<400>  8

Ile Leu Gly Gly Arg Glu Ala Glu Ala His Ala Arg Pro Tyr Met Ala

1               5                   10                  15

Ser Val Gln Leu Asn Gly Ala His Leu Cys Gly Gly Val Leu Val Ala
            20                  25                  30

Glu Gln Trp Val Leu Ser Ala Ala His Cys Leu Glu Asp Ala Ala Asp
        35                  40                  45

Gly Lys Val Gln Val Leu Leu Gly Ala His Ser Leu Ser Gln Pro Glu
    50                  55                  60

Pro Ser Lys Arg Leu Tyr Asp Val Leu Arg Ala Val Pro His Pro Asp
65                  70                  75                  80

Ser Gln Pro Asp Thr Ile Asp His Asp Leu Leu Leu
                85                  90